(19) **Europäisches Patentamt / European Patent Office / Office européen des brevets**

(11) **EP 3 747 475 A1**

(12) # EUROPEAN PATENT APPLICATION
published in accordance with Art. 153(4) EPC

(43) Date of publication:
09.12.2020 Bulletin 2020/50

(21) Application number: 19748378.7

(22) Date of filing: 29.01.2019

(51) Int Cl.:
*A61L 27/22* (2006.01)     *A61C 8/00* (2006.01)
*A61L 27/24* (2006.01)     *C07K 14/75* (2006.01)
*C07K 14/78* (2006.01)     *C12N 15/12* (2006.01)
*C12P 21/02* (2006.01)

(86) International application number:
PCT/JP2019/002869

(87) International publication number:
WO 2019/151205 (08.08.2019 Gazette 2019/32)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 30.01.2018 JP 2018013283

(71) Applicant: FUJIFILM Corporation
Tokyo 106-8620 (JP)

(72) Inventors:
• HIRATSUKA, Takahiro
Ashigarakami-gun, Kanagawa 258-8577 (JP)
• AZUMA, Akihiko
Ashigarakami-gun, Kanagawa 258-8577 (JP)
• KAWASE, Tomoyuki
Niigata-shi, Niigata 950-2181 (JP)

(74) Representative: Hoffmann Eitle
Patent- und Rechtsanwälte PartmbB
Arabellastraße 30
81925 München (DE)

(54) **FIBRIN COMPOSITION, BASE MATERIAL FOR REGENERATIVE MEDICINE, METHOD FOR PRODUCING FIBRIN COMPOSITION, AND KIT**

(57)   An object of the present invention is to provide a fibrin composition manufactured by a method as a substitute for a method for manufacturing platelet-rich fibrin (PRF) triggered by the activation of a coagulation factor, a substrate for regenerative medicine using the fibrin composition, a method for manufacturing a fibrin composition, and a kit for being used in the method. According to the present invention, there is provided a fibrin composition including blocks, which each include a protein having repetitive Arg-Gly-Asp sequences derived from human collagen, fibrin, and platelets.

FIG. 1A

FIG. 1B

FIG. 1C

```
SEQUENCE LISTING
<110>  FUJIFILM Corporation
<120>  Fibrin composition, base material for regenerative medicine, method
for production of fibrin composition, and kit
<130>\201@17F03563W1
<160>  1
<170>  PatentIn version 3.5
<210>  1
<211>  571
<212>  PRT
<213>  Recombinant
<400>  1
Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
                20                  25                  30
Arg Gly Asp Ala Gly Pro Lys Gly Ala Asp Gly Ala Pro Gly Ala Pro
            35                  40                  45
Gly Leu Gln Gly Met Pro Gly Glu Arg Gly Ala Ala Gly Leu Pro Gly
        50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
            100                 105                 110
Asp Ala Gly Pro Lys Gly Ala Asp Gly Ala Pro Gly Lys Asp Gly Val
            115                 120                 125
Arg Gly Leu Ala Gly Pro Ile Gly Pro Pro Gly Pro Ala Gly Ala Pro
            130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
                165                 170                 175
Asp Gly Ala Pro Gly Lys Asp Gly Val Arg Gly Leu Ala Gly Pro Pro
                180                 185                 190
Gly Ala Pro Gly Leu Gln Gly Ala Pro Gly Leu Gln Gly Met Pro Gly
                195                 200                 205
Glu Arg Gly Ala Ala Gly Leu Pro Gly Pro Lys Gly Glu Arg Gly Asp
        210                 215                 220
Ala Gly Pro Lys Gly Ala Asp Gly Ala Pro Gly Ala Pro Gly Leu Gln
225                 230                 235                 240
Gly Met Pro Gly Glu Arg Gly Ala Ala Gly Leu Pro Gly Pro Lys Gly
                245                 250                 255
Glu Arg Gly Asp Ala Gly Pro Lys Gly Ala Asp Gly Ala Pro Gly Lys
                260                 265                 270
Asp Gly Val Arg Gly Leu Ala Gly Pro Ile Gly Pro Pro Gly Glu Arg
            275                 280                 285
Gly Ala Ala Gly Leu Pro Gly Pro Lys Gly Glu Arg Gly Asp Ala Gly
            290                 295                 300
Pro Lys Gly Ala Asp Gly Ala Pro Gly Lys Asp Gly Val Arg Gly Leu
305                 310                 315                 320
Ala Gly Pro Ile Gly Pro Pro Gly Pro Ala Gly Ala Pro Gly Ala Pro
                325                 330                 335
Gly Leu Gln Gly Met Pro Gly Glu Arg Gly Ala Ala Gly Leu Pro Gly
                340                 345                 350
Pro Lys Gly Glu Arg Gly Asp Ala Gly Pro Lys Gly Ala Asp Gly Ala
            355                 360                 365
Pro Gly Lys Asp Gly Val Arg Gly Leu Ala Gly Pro Pro Gly Ala Pro
            370                 375                 380
Gly Leu Gln Gly Ala Pro Gly Leu Gln Gly Met Pro Gly Glu Arg Gly
385                 390                 395                 400
Ala Ala Gly Leu Pro Gly Pro Lys Gly Glu Arg Gly Asp Ala Gly Pro
```

```
                        405                      410                      415
    Lys Gly Ala Asp Gly Ala Pro Gly Ala Pro Gly Leu Gln Gly Met Pro
                420                      425                  430
    Gly Glu Arg Gly Ala Ala Gly Leu Pro Gly Pro Lys Gly Glu Arg Gly
            435                      440                  445
    Asp Ala Gly Pro Lys Gly Ala Asp Gly Ala Pro Gly Lys Asp Gly Val
        450                      455                  460
    Arg Gly Leu Ala Gly Pro Ile Gly Pro Pro Gly Glu Arg Gly Ala Ala
    465                  470                  475                  480
    Gly Leu Pro Gly Pro Lys Gly Glu Arg Gly Asp Ala Gly Pro Lys Gly
                    485                  490                  495
    Ala Asp Gly Ala Pro Gly Lys Asp Gly Val Arg Gly Leu Ala Gly Pro
                500                      505                  510
    Ile Gly Pro Pro Gly Pro Ala Gly Ala Pro Gly Ala Pro Gly Leu Gln
                515                      520                  525
    Gly Met Pro Gly Glu Arg Gly Ala Ala Gly Leu Pro Gly Pro Lys Gly
        530                      535                  540
    Glu Arg Gly Asp Ala Gly Pro Lys Gly Ala Asp Gly Ala Pro Gly Lys
    545                      550                  555                  560
    Asp Gly Val Arg Gly Leu Ala Gly Pro Pro Gly
                    565                      570
```

**Description**

Field of the Invention

The present invention relates to a fibrin composition including blocks, which each include a protein having an amino acid sequence derived from human collagen, fibrin, and platelets. Furthermore, the present invention relates to a substrate for regenerative medicine including the fibrin composition, a method for manufacturing a fibrin composition, and a kit.

Description of the Related Art

In regenerative medicine such as alveolar bone reconstruction and periodontal regeneration therapy, a platelet-concentrated material is used. As the platelet-concentrated material, there are platelet-rich plasma (PRP) prepared from blood including an anticoagulant and platelet-rich fibrin (PRF) prepared from blood including no anticoagulant. PRF isolated and prepared from autologous blood was developed in the early 2000s, and has become widespread rapidly. PRF is considered to exhibit therapeutic effects by abundant growth factors derived from platelets and fibrin just as PRP. In order to prepare PRP, it is necessary to perform blood collection using an anticoagulant and the subsequent complicated operations such as centrifugation and pipetting. In contrast, PRF can be prepared simply by centrifugation, which is an advantage of PRF.

Patent Document 1 describes a method for manufacturing a low-degradable fibrin gel membrane for regenerative therapy and a device for manufacturing the membrane. Patent Document 2 describes a fibrin gel rolling device capable of uniformly and thinly rolling a fibrin gel without damaging it. Patent Document 3 describes a preparation of a blood composition that is enriched with platelets and/or growth factors and contains a gelated protein. Patent Document 4 describes a tube for generating platelet-rich fibrin, in which the surface of the tube contacting blood during centrifugation is constituted with pure titanium or a titanium alloy.

Conventionally, for preparing PRF, blood collection tubes made of glass have been generally used. By the contact with a glass surface, the coagulation factor XII is activated first, then the endogenous coagulation system is subsequently activated, and finally, fibrinogen is converted into fibrin by thrombin, whereby fibrin clots are formed. The blood collection tubes made of glass are used for this reason. However, the manufacturing of glass blood collection tubes has been discontinued due to the influence of the recent ecological concerns. Under such circumstances, in some cases, PRF is prepared using a plastic blood collection tube including a film which includes silica for a blood coagulation activity test. However, because the plastic blood collection tube is not manufactured for the re-administration of the collected blood components, biosafety thereof is not guaranteed.

Prior Art Documents

Patent Documents

Patent Document 1: JP2015-167606A
Patent Document 2: WO2012/102094A
Patent Document 3: JP2016-528225A
Patent Document 4: JP2014-531467A:

**SUMMARY OF THE INVENTION**

An object of the present invention is to provide a fibrin composition, which is manufactured by a method as a substitute for a method for manufacturing platelet-rich fibrin (PRF) triggered by the activation of a coagulation factor, and a substrate for regenerative medicine using the fibrin composition. Another object of the present invention is to provide a method for manufacturing a fibrin composition, which is a substitute for a method for manufacturing PRF triggered by the activation of a coagulation factor, and a kit for being used in the method.

In order to achieve the above objects, the inventors of the present invention made an attempt at performing a method using a human collagen-like artificial protein, as a method substituting the method for manufacturing a fibrin composition triggered by the activation of a coagulation factor. That is, by using a blood collection tube, which was prepared by adding blocks each including a protein having repetitive Arg-Gly-Asp sequences derived from human collagen to a plastic blood collection tube that does not promote blood coagulation, the inventors manufactured a fibrin composition. As a result, the inventors of the present invention have proved that the fibrin composition, which includes blocks each including a protein having repetitive Arg-Gly-Asp sequences derived from human collagen, fibrin, and platelets, markedly promotes bone regeneration in a living body. The present invention has been accomplished based on the above finding.

That is, according to the present invention, the following inventions are provided.

(1) A fibrin composition including blocks, which each include a protein having repetitive Arg-Gly-Asp sequences derived from human collagen, fibrin, and platelets.

(2) The fibrin composition described in (1), in which the blocks are in the form of granules.

(3) The fibrin composition described in (1) or (2), in which the blocks are in the form of granules obtained by sieving blocks obtained by pulverizing a porous material of the protein.

(4) The fibrin composition described in (2) or (3), in which the granules have such a size that the granules pass through a 1,000 μm sieve and remain on a 100 μm sieve.

(5) The fibrin composition described in (2) or (3), in which the granules have such a size that the granules pass through a 710 μm sieve and remain on a 500 μm sieve.

(6) The fibrin composition described in any one of (2) to (5), in which a porosity of the granules is 70% to 95%.

(7) The fibrin composition described in any one of (1) to (6), in which the protein is a recombinant peptide.

(8) The fibrin composition described in (7), in which the recombinant peptide is represented by the following formula.

Formula:          A-[(Gly-X-Y)n]m-B

In the formula, A represents any amino acid or amino acid sequence, B represents any amino acid or amino acid sequence, n pieces of X each independently represent any amino acid, n pieces of Y each independently represent any amino acid, n represents an integer of 3 to 100, m represents an integer of 2 to 10, and n pieces of Gly-X-Y may be the same as or different from each other.

(9) The fibrin composition described in (7) or (8), in which the recombinant peptide is any of

a peptide consisting of an amino acid sequence described in SEQ ID NO: 1;

a peptide having an amino acid sequence, which is obtained by the deletion, substitution, or addition of one or several amino acids in the amino acid sequence described in SEQ ID NO: 1, and having biocompatibility; or

a peptide having an amino acid sequence, which shares sequence identity equal to or higher than 80% with the amino acid sequence described in SEQ ID NO: 1, and having biocompatibility.

(10) The fibrin composition described in any one of (1) to (9), in which in the blocks, the protein is crosslinked by heat, ultraviolet rays, or an enzyme.

(11) A substrate for regenerative medicine, including the fibrin composition described in any one of (1) to (10).

(12) A method for manufacturing a fibrin composition, including a step of mixing blocks, which each include a protein having repetitive Arg-Gly-Asp sequences derived from human collagen, with blood: and

a step of subjecting the obtained mixture to centrifugation.

(13) The method described in (12), in which the blocks are in the form of granules.

(14) The method described in (12) or (13), in which the blocks are in the form of granules obtained by pulverizing a porous material of the protein.

(15) The method described in (13) or (14), in which the granules have such a size that the granules pass through a 1,000 μm sieve and remain on a 100 μm sieve.

(16) The method described in (13) or (14), in which the granules have such a size that the granules pass through a 710 μm sieve and remain on a 500 μm sieve.

(17) The method described in any one of (13) to (16), in which a porosity of the granules is 70% to 95%.

(18) The method described in any one of (12) to (17), in which the protein is a recombinant peptide.

(19) The method described in (18), in which the recombinant peptide is represented by the following formula.

Formula:          A-[(Gly-X-Y)n]m-B

In the formula, A represents any amino acid or amino acid sequence, B represents any amino acid or amino acid sequence, n pieces of X each independently represent any amino acid, n pieces of Y each independently represent any amino acid, n represents an integer of 3 to 100, m represents an integer of 2 to 10, and n pieces of Gly-X-Y may be the same as or different from each other.

(20) The method described in (18) or (19), in which the recombinant peptide is any of

a peptide consisting of an amino acid sequence described in SEQ ID NO: 1;

a peptide having an amino acid sequence, which is obtained by the deletion, substitution, or addition of one or several amino acids in the amino acid sequence described in SEQ ID NO: 1, and having biocompatibility; or

a peptide having an amino acid sequence, which shares sequence identity equal to or higher than 80% with the amino acid sequence described in SEQ ID NO: 1, and having biocompatibility.

(21) The method described in any one of (12) to (20), in which in the blocks, the protein is crosslinked by heat, ultraviolet rays, or an enzyme.

(22) The method described in any one of (12) to (21), in which the blood is whole blood.

(23) The method described in any one of (12) to (22), in which a mass ratio of the blocks to the blood is 1:500 to 1:100.

(24) A kit for being used in the method for manufacturing a fibrin composition described in any one of (12) to (23), including blocks, which each include a protein having repetitive Arg-Gly-Asp sequences derived from human collagen, and a plastic tube.

According to the present invention, there is provided a fibrin composition, which is manufactured by a method as a substitute for a method for manufacturing PRF triggered by the activation of a coagulation factor, and a substrate for regenerative medicine using the fibrin composition. Furthermore, according to the present invention, there are provided a method for manufacturing a fibrin composition, which is a substitute for a method for manufacturing PRF triggered by the activation of a coagulation factor, and a kit for being used in the method.

## BRIEF DESCRIPTION OF THE DRAWINGS

Figs. 1A to 1C show the states of a fibrin composition observed with the unaided eye.

Figs. 2A to 2C shows images of the fibrin composition captured using a scanning electron microscope.

Fig. 3 shows images of the fibrin composition captured using a scanning electron microscope.

Fig. 4 shows the results of comparison of bone regeneration effects in cranial bond-deficient mouse models.

## DESCRIPTION OF THE PREFERRED EMBODIMENTS

Hereinafter, embodiments for carrying out the present invention will be specifically described.

[Fibrin composition]

The fibrin composition according to an embodiment of the present invention is a fibrin composition including blocks, which each include a protein having repetitive Arg-Gly-Asp sequences derived from human collagen, fibrin, and platelets.

The fibrin composition according to the embodiment of the present invention can be manufactured by adding blocks, which each include a protein having repetitive Arg-Gly-Asp sequences derived from human collagen, and blood to a plastic tube. The fibrin composition according to the embodiment of the present invention can be manufactured without using a blood collection tube made of glass. Therefore, from the viewpoint of costs and from the viewpoint of international ecological policies, the fibrin composition is extremely advantageous.

The fibrin composition according to the embodiment of the present invention is expected to be capable of bring about bone regeneration effects by a synergism between the blocks, which each include protein having repetitive Arg-Gly-Asp sequences derived from human collagen, and platelet-rich fibrin (PRF).

The fibrin composition according to the embodiment of the present invention makes it possible to transplant a complex of the blocks which each include a protein having repetitive Arg-Gly-Asp sequences derived from human collagen, fibrin, and platelets as a single unit. Therefore, the fibrin composition makes it possible to save time and to reduce the variation in the effect resulting from the difference in skills between operators.

<Protein having repetitive Arg-Gly-Asp sequences derived from human collagen>

In one molecule of the protein having repetitive Arg-Gly-Asp sequences derived from human collagen, there are two or more sequences of cell adhesion signals represented by the Arg-Gly-Asp sequence.

The protein having repetitive Arg-Gly-Asp sequences derived from human collagen is preferably a recombinant peptide.

As the protein having repetitive Arg-Gly-Asp sequences derived from human collagen, it is possible to use recombinant gelatin having an amino acid sequence derived from a partial amino acid sequence of collagen. For example, it is possible to use the proteins described in EP1014176B, US6992172B, WO2004/085473A, WO2008/103041A, and the like, but the present invention is not limited to these. As the recombinant gelatin used in the present invention, recombinant gelatin according to the following embodiment is preferable.

Recombinant gelatin is excellently biocompatible due to the performance thereof inherent to natural gelatin, has no concern of bovine spongiform encephalopathy (BSE) because the gelatin is not derived from nature, and is excellently noninfective. In addition, recombinant gelatin is more uniform than natural gelatin, and the sequence thereof is determined. Therefore, by means of crosslinking and the like, it is possible to precisely design the strength and degradability of the recombinant gelatin with less variation.

The molecular weight of the recombinant gelatin is not particularly limited, but is preferably equal to or greater than 2,000 and equal to or smaller than 100,000 (equal to or greater than 2 kDa (kilodalton) and equal to or smaller than 100 kDa), more preferably equal to or greater than 2,500 and equal to or smaller than 95,000 (equal to or greater than 2.5 kDa and equal to or smaller than 95 kDa), even more preferably equal to or greater than 5,000 and equal to or smaller than 90,000 (equal to or greater than5 kDa and equal to or smaller than 90 kDa), and most preferably equal to or greater than 10,000 and equal to or smaller than 90,000 (equal to or greater than 10 kDa and equal to or smaller than90 kDa).

It is preferable that the recombinant gelatin has repeat sequences represented by Gly-X-Y distinct to collagen. The plurality of Gly-X-Y sequences may be the same as or different from each other. In Gly-X-Y, Gly represents glycine, and X and Y represent any amino acid (preferably, any amino acid other than glycine). The sequence represented by Gly-X-Y distinct to collagen is an extremely specific partial structure compared with other proteins in the composition and sequence of amino acids of gelatin collagen. In this part, glycine accounts for about one third of the whole peptide, and in the amino acid sequence, glycine is one of the three repetitive amino acids. Glycine is the simplest amino acid, has little restriction on the arrangement of the molecular chain thereof, and greatly contributes to the regeneration of the helical structure during gelation. The amino acids represented by X and Y include a large amount of imino acid (proline or oxyproline), and preferably account for 10% to 45% of the whole peptide. The proportion of the Gly-X-Y repetitive structures in the amino acids constituting the sequence of the recombinant gelatin is preferably equal to or higher than 80%, more preferably equal to or higher than 95%, and most preferably equal to or higher than 99%.

Generally, in gelatin, a charged polar amino acid and an uncharged polar amino acid are present at a ratio of 1:1. Specifically, the polar amino acid refers to cysteine, aspartic acid, glutamic acid, histidine, lysine, asparagine, glutamine, serine, threonine, tyrosine, and arginine. Among these, cysteine, asparagine, glutamine, serine, threonine, and tyrosine are uncharged polar amino acids. In the recombinant gelatin used in the present invention, the proportion of polar amino acids in all constituent amino acids is 10% to 40%, and preferably 20% to 30%. Furthermore, it is preferable that the proportion of uncharged amino acids in the polar amino acids is equal to or higher than 5% and less than 20%, and more preferably equal to or higher than 5% and less 10%. Furthermore, it is preferable that the sequence of the recombinant gelatin does not have any one amino acid and more preferably two or more amino acids among serine, threonine, asparagine, tyrosine, and cysteine.

Regarding the arrangement of RGD sequences in the recombinant gelatin used in the present invention, the number of amino acids between RGD is preferably nonuniform in a range of 0 to 100, and more preferably nonuniform in a range of 25 and 60.

From the viewpoint of cell adhesion proliferation properties, the number of these minimum amino acid sequences included in one molecule of the protein is preferably 3 to 50, more preferably 4 to 30, particularly preferably 5 to 20, and most preferably 12.

In the recombinant gelatin used in the present invention, the ratio of the RGD motif to the total number of amino acids is preferably at least 0.4%. In a case where the recombinant gelatin includes 350 or more amino acids, it is preferred that each stretch of 350 amino acids includes at least one RGD motif. The ratio of the RGD motif to the total number of amino acids is more preferably at least 0.6%, even more preferably at least 0.8%, still more preferably at least 1.0%, particularly preferably at least 1.2%. %, and most preferably at least 1.5%. In the recombinant peptide, the number of RGD motifs per 250 amino acids is preferably at least 4, more preferably at least 6, even more preferably at least 8, and particularly preferably equal to or greater than 12 and equal to or smaller than 16. The ratio of 0.4% of the RGD motif means that there is at least one RGD sequence per 250 amino acids. Because the number of RGD motifs is an integer, gelatin consisting of 251 amino acids should have at least two RGD sequences to satisfy the feature of 0.4%. The recombinant gelatin according to the embodiment of the present invention preferably has at least two RGD sequences per 250 amino acids, more preferably has at least three RGD sequences per 250 amino acids, and even more preferably has at least four RGD sequences per 250 amino acids. In another embodiment, the recombinant gelatin according to the embodiment of the present invention preferably has at least four RGD motifs, more preferably has at least six RGD motifs, even more preferably has at least eight RGD motifs, and particularly preferably has 12 to 16 RGD motifs.

The recombinant gelatin may be partially hydrolyzed.

It is preferable that the recombinant gelatin used in the present invention is represented by Formula 1: A-[(Gly-X-Y)$_n$]$_m$-B. n pieces of X each independently represent any amino acid, and n pieces of Y each independently represent any amino acid. m preferably represents an integer of 2 to 10, and more preferably represents an integer of 3 to 5. n is preferably an integer of 3 to 100, more preferably an integer of 15 to 70, and most preferably an integer of 50 to 65. A represents any amino acid or amino acid sequence, and B represents any amino acid or amino acid sequence. n pieces of Gly-X-Y may be the same as or different from each other.

The recombinant gelatin used in the present invention is more preferably represented by Gly-Ala-Pro-[(Gly-X-Y)$_{63}$]$_3$-Gly (in the formula, 63 Xs each independently represent any amino acid, 63 Ys each independently represents any amino acid, and 63 Gly-X-Y sequences may be the same as or different from each other).

It is preferable to bind a plurality of sequence units of naturally occurring collagen to the repeating unit. The naturally occurring collagen mentioned here is not limited as long as it is naturally occurring collagen, but is preferably type I, type II, type III, type IV, or type V collagen. Among these, type I, type II, or type III collagen is more preferable. According to another embodiment, the source of the collagen is preferably human beings, cows, pigs, or mice or rats, and more preferably human beings.

The isoelectric point of the recombinant gelatin used in the present invention is preferably 5 to 10, more preferably 6 to 10, and even more preferably 7 to 9.5. The method for measuring the isoelectric point of the recombinant gelatin is not limited as long as it is a known method capable of measuring isoelectric point. For example, by isoelectric point electrophoresis (see Maxey, CR (1976; Phitogr. Gelatin 2, Editor Cox, PJ Academic, London, Engl.), the isoelectric point can be measured by means of measuring pH after passing a 1% by weight gelatin solution through a mixed crystal column of cation and anion exchange resins.

It is preferable that the recombinant gelatin is not deaminated.

It is preferable that the recombinant gelatin does not have a telopeptide.

It is preferable that the recombinant gelatin is substantially a pure polypeptide prepared by a nucleic acid encoding an amino acid sequence.

As the recombinant gelatin used in the present invention, any of the following peptides is preferable.

(1) Peptide consisting of an amino acid sequence described in SEQ ID NO: 1
(2) Peptide having an amino acid sequence, which is obtained by the deletion, substitution, or addition of one or several amino acids in the amino acid sequence described in SEQ ID NO: 1, and having biocompatibility
(3) Peptide having an amino acid sequence, which shares sequence identity equal to or higher than 80% (more preferably equal to or higher than 85%, even more preferably equal to or higher than 90%, particularly preferably equal to or higher than 95%, and most preferably equal to or higher than 98%) with the amino acid sequence described in SEQ ID NO: 1, and having biocompatibility

In the present invention, the sequence identity refers to a value calculated by the following formula.

$$\text{Sequence identity } (\%) = [(\text{number of identical residues})/(\text{length of longer alignment})] \times 100$$

The sequence identity between two amino acid sequences can be determined by any method known to those skilled in the art, and can also be determined using the BLAST ((Basic Local Alignment Search Tool)) program (J. Mol. Biol. 215: 403-410, 1990) and the like. "Length of longer alignment" of the denominator means that in a case where two alignments are compared with each other, the length of a longer alignment is used as the denominator.

"One or several amino acids" in "amino acid sequence obtained by the deletion, substitution, or addition of one or several amino acids" preferably means 1 to 20 amino acids, more preferably means 1 to 10 amino acids, even more preferably means 1 to 5 amino acids, and particularly preferably means 1 to 3 amino acids.

The value of "1/IOB" which is a value of hydrophilicity of "protein having repetitive Arg-Gly-Asp sequences derived from human collagen" used in the present invention is preferably 0 to 1.0. The value of 1/IOB is more preferably 0 to 0.6, and even more preferably 0 to 0.4. IOB is an index of hydropathicity based on the organic conception diagram showing polarity/non-polarity of organic compounds that was suggested by Atsushi Fujita. Details of IOB are described, for example, in "Pharmaceutical Bulletin", vol. 2, 2, pp. 163-173 (1954), "Scope of Chemistry", vol. 11, 10, pp.719-725 (1957), "Fragrance Journal", vol. 50, pp. 79-82 (1981), and the like. In brief, methane (CH$_4$) is regarded as the origin of all organic compounds, all other compounds are regarded as derivatives of methane, and a certain numerical value is assigned to the number of carbon atoms, the substituent, the transformative portion, the ring, and the like of the compounds. The scores are added up to determine an organic value (OV) and an inorganic value (IV), and a graph is created by plotting the organic value on the X-axis and the inorganic value on the Y-axis. IOB in the organic conception diagram refers to a ratio of the inorganic value (IV) to the organic value (OV) in the organic conception diagram, that is, "inorganic value (IV)/organic value (OV)". For details of the organic conception diagram, see "New Edition of Organic Conception Diagram-Fundamentals and Applications-" (Yoshiki Koda et al., SANKYO SHUPPAN Co., Ltd., 2008). In the present specification, hydropathicity is represented by "1/IOB" value which is the reciprocal of IOB. The smaller the "1/IOB" value (the closer the "1/IOB" value to 0), the higher the hydrophilicity.

In a case where the "1/IOB" value of "protein having repetitive Arg-Gly-Asp sequences derived from human collagen" used in the present invention is within the above range, the hydrophilicity is high, and the water absorbing properties are improved.

For the protein used in the present invention, which has repetitive Arg-Gly-Asp sequences derived from human collagen, a hydropathicity index represented by a Grand average of hydropathicity (GRAVY) value is preferably equal to or lower than 0.3 and equal to or higher than -9.0, and more preferably equal to or lower than 0.0 and equal to or higher than -7.0. The Grand average of hydropathicity (GRAVY) value can be obtained by the methods described in "Gasteiger E., Hoogland C., Gattiker A., Duvaud S., Wilkins M.R., Appel R.D., Bairoch A.;Protein Identification and Analysis Tools on the ExPASy Server;(In) John M. Walker (ed): The Proteomics Protocols Handbook, Humana Press (2005). pp. 571-607" and "Gasteiger E., Gattiker A., Hoogland C., Ivanyi I., Appel R.D., Bairoch A.; ExPASy: the proteomics server for in-depth protein knowledge and analysis.; Nucleic Acids Res. 31:3784-3788 (2003)".

In a case where the GRAVY value is within the above range, the hydrophilicity is high, and the water absorbing properties are improved.

The protein used in the present invention, which has repetitive Arg-Gly-Asp sequences derived from human collagen, can be manufactured by gene recombination technologies known to those skilled in the art. For example, the protein can be manufactured based on the methods described in EP1014176A2, US6992172B, WO2004/085473A, WO2008/103041A, and the like. Specifically, genes encoding an amino acid sequence of a predetermined protein are obtained and incorporated into an expression vector, thereby preparing a recombinant expression vector. The vector is introduced into an appropriate host, thereby preparing a transformant. By culturing the obtained transformant in an appropriate medium, recombinant gelatin is produced. Therefore, by collecting the produced recombinant gelatin from the culture, it is possible to prepare the protein used in the present invention, which has repetitive Arg-Gly-Asp sequences derived from human collagen.

<Crosslinking>

The protein used in the present invention, which has repetitive Arg-Gly-Asp sequences derived from human collagen, may be crosslinked or non-crosslinked, but is preferably crosslinked. In a case where the crosslinked protein is used, it is possible to obtain an effect of preventing the protein from being rapidly degraded at the time of culturing the protein in a medium or at the time of transplanting the protein into a living body. As general crosslinking methods, thermal crosslinking, crosslinking by aldehydes (for example, formaldehyde, glutaraldehyde, and the like), crosslinking by a condensing agent (such as carbodiimide or cyanamide), enzymatic crosslinking, photocrosslinking, ultraviolet crosslinking, hydrophobic interaction, hydrogen bonding, ionic interaction, and the like are known. These crosslinking methods can also be used in the present invention. As the crosslinking method used in the present invention, thermal crosslinking, ultraviolet crosslinking, or enzyme crosslinking is preferable, and thermal crosslinking is particularly preferable.

In a case where the protein is crosslinked using an enzyme, the enzyme is not particularly limited as long as it acts to crosslink protein materials with each other. The protein can be crosslinked preferably using transglutaminase or laccase, and most preferably using transglutaminase. Specific examples of the protein that is enzymatically crosslinked by transglutaminase are not particularly limited as long as the protein has a lysine residue and a glutamine residue. The transglutaminase may be derived from mammals or microorganisms. Specifically, examples thereof include an ACTIVA series manufactured by AJINOMOTO CO., INC., mammal-derived transglutaminase marketed as a reagent such as guinea pig liver-derived transglutaminase, goat-derived transglutaminase, and rabbit-derived transglutaminase manufactured by Oriental Yeast Co., ltd., Upstate USA Inc., Biodesign International, and the like, human-derived blood coagulation factors (Factor XIIIa, Haematologic Technologies, Inc.), and the like.

The reaction temperature at the time of performing crosslinking (for example, thermal crosslinking) is not particularly limited as long as crosslinks can be formed. However, the reaction temperature is preferably-100°C to 500°C, more preferably 0°C to 300°C, even more preferably 50°C to 300°C, particularly preferably 100°C to 250°C, and most preferably 120°C to 200°C.

<Block>

In the present invention, blocks (lumps) each including the aforementioned protein having repetitive Arg-Gly-Asp sequences derived from human collagen is used.

The form of the blocks is not particularly limited. For example, the blocks may be amorphous, spherical, particle-like (granular), powdery, porous, fibrous, spindle-shaped, or flat and sheet-like, preferably amorphous, spherical, particle-like (granular), powdery, or porous, and particularly preferably granular. "Amorphous" means that the surface shape is non-uniform, and refers to a substance having irregularities such as a rock. In addition, the shapes exemplified above are not separate shapes. For example, sometimes "amorphous" may be an example of a subordinate concept of "particle-like (granular)".

The shape of the blocks in the present invention is not particularly limited, but the tapped density of the blocks is preferably equal to or higher than 10 mg/cm$^3$ and equal to or lower than 500 mg/cm$^3$, more preferably equal to or higher than 20 mg/cm$^3$ and equal to or lower than 400 mg/cm$^3$, even more preferably equal to or higher than 40 mg/cm$^3$ and equal to or lower than 220 mg/cm$^3$, and particularly preferably equal to or higher than 50 mg/cm$^3$ and equal to or lower than 150 mg/cm$^3$.

The tapped density is a value showing how many blocks can densely fill up a certain volume. A low tapped density shows that the blocks cannot densely fill up the volume, in other words, each of the blocks has a complicated structure. The tapped density of the blocks is considered to represent the complexity of the surface structure of the blocks and the amount of voids formed in a case where the blocks are gathered as an aggregate. The lower the tapped density, the more the voids between the blocks and the larger the area into which cells are engrafted. In addition, in a case where the tapped density is not extremely low, the blocks can be appropriately located between cells.

The tapped density mentioned in the present specification is not particularly limited, and can be measured as follows. For measurement, a container (cylindrical container having a diameter of 6 mm, a length of 21.8 mm, and a volume of 0.616 cm$^3$) is prepared (hereinafter, the container will be referred to as cap). First, the mass of the cap is measured. Then, a funnel is attached to the cap, and the blocks are poured into the cap from the funnel such that the blocks are accumulated in the cap. After the cap is filled with a sufficient amount of blocks, the portion of the cap is tapped 200 times on a hard object such as a desk, the funnel is detached, and the blocks are leveled off with a spatula. The mass of the cap fully packed with the blocks is measured. By calculating the mass of the blocks from the difference between the mass of the cap and the mass of the cap with blocks and dividing the mass of the blocks by the volume of the cap, the tapped density can be determined.

The degree of crosslinking of the blocks (the number of crosslinks per molecule) is not particularly limited, but is preferably equal to or higher than 2, more preferably equal to or higher than 2 and equal to or lower than 30, even more preferably equal to or higher than 4 and equal to or lower than 25, and particularly preferably equal to or higher than 4 and equal to or lower than 22.

The method for measuring the degree of crosslinking of the blocks (the number of crosslinks per molecule) is not particularly limited. In a case where the protein is CBE3, the degree of crosslinking can be measured, for example, by a TNBS (2,4,6-trinitrobenzenesulfonic acid) method in Examples which will be described later. Specifically, a sample is prepared by mixing together the blocks, an aqueous solution of NaHCO$_3$, and an aqueous solution of TNBS, allowing these to react for 3 hours at 37°C, and then interrupting the reaction. Furthermore, a blank is prepared by mixing together the blocks, an aqueous solution of NaHCO$_3$, and an aqueous solution of TNBS, and then immediately interrupting the reaction. The sample and the blank are diluted with pure water, and an absorbance (345 nm) thereof is measured. Thereafter, by the following (Equation 2) and (Equation 3), the degree of crosslinking (number of crosslinks per molecule) can be calculated.

$$(\text{Equation 2}) \ (As - Ab)/14600 \times V/w$$

(Equation 2) shows the amount of lysine (molar equivalent) per 1 g of the blocks.

(In the equation, As represents the absorbance of the sample, Ab represents the absorbance of the blank, V represents the amount of the reaction solution (g), and w represents the mass of the blocks (mg).)

$$(\text{Equation 3}) \ 1 - (\text{Sample (Equation 2)/uncrosslinked protein (Equation 2)}) \times 34$$

(Equation 3) shows the number of crosslinks per molecule.

The water absorption rate of the blocks is not particularly limited, but is preferably equal to or higher than 300%, more preferably equal to or higher than 400%, even more preferably equal to or higher than 500%, particularly preferably equal to or higher than 600%, and most preferably equal to or higher than 700%. The upper limit of the water absorption rate is not particularly limited, but is generally equal to or lower than 4,000% or equal to or lower than 2,000%.

The method for measuring the water absorption rate of the blocks is not particularly limited. For example, the water absorption rate can be measured by the method in Examples which will be described later. Specifically, a 3 cm × 3 cm nylon mesh bag is filled with about 15 mg of the blocks at 25°C, caused to swell in deionized water for 2 hours, and then air-dried for 10 minutes. By measuring the mass at each stage, the water absorption rate can be determined according to (Equation 4).

(Equation 4)

$$\text{Water absorption rate} = (w2 - w1 - w0)/w0$$

(In the equation, w0 represents the mass of the material before absorbing water, w1 represents the mass of the empty bag after absorbing water, and w2 represents the total mass of the bag including the material after absorbing water.)

The blocks used in the present invention are preferably in the form of granules obtained by sieving blocks obtained by pulverizing a porous material of a protein. The blocks used in the present invention are more preferably granules having a desired size, which are obtained by crosslinking blocks obtained by pulverizing a porous material of a protein so as to obtain crosslinked blocks and then sieving the obtained crosslinked blocks.

The granules preferably have such a size that the granules pass through a 1,000 μm sieve and remain on a 100 μm sieve, and more preferably have such a size that the granules pass through a 710 μm sieve and remain on a 500 μm sieve.

The granules are sieved using a test sieve that meets the ISO3310 standard by the sieving method described in the second method of The Japanese Pharmacopoeia, 16th edition, section 3.04. That is, an operation of shaking the granules for 5 minutes is intermittently performed a plurality of times, and at a point in time when the ratio of the mass of a group of particles remaining on the sieve after the shaking to the mass of a group of particles on the sieve before the shaking becomes equal to or lower than 5%, the operation is ended. In the present invention, "passing" means that the ratio of particles remaining on the sieve at the ending time to the total mass of the particles not yet being sieved is equal to or lower than 10% by mass, and the term "remaining" means that the ratio of particles remaining on the sieve at the ending time to the total mass of the particles not yet being sieved is equal to or higher than 95% by mass.

In a case where the blocks are granules, the porosity of the granules is preferably 70% to 95%, and more preferably 80% to 90%.

The porosity (P) of the granules can be calculated by the following equation by using bulk density (p) and true density (pc).

$$P = 1 - \rho/\rho c \ (\%)$$

The bulk density (p) can be calculated from dry weight and volume, and the true density (pc) can be determined by a Hubbard specific gravity bottle method.

<Method for manufacturing blocks>

The method for manufacturing the blocks is not particularly limited, and the blocks can be obtained by the following method for example.

One example of the method for manufacturing the blocks may include (a) preparing a protein solution including a protein, which has repetitive Arg-Gly-Asp sequences derived from human collagen, dissolved in an aqueous medium (hereinafter, referred to as protein solution preparation step), (b) freeze-drying the protein solution so as to obtain a freeze-dried material (hereinafter, referred to as freeze-drying step), (c) pulverizing the freeze-dried material of the protein so as to obtain a pulverized material (hereinafter, referred to as pulverization step), and (d) crosslinking the pulverized material so as to obtain a substrate (hereinafter, referred to as crosslinking step).

The aqueous medium of the protein solution prepared by the protein solution preparation step is not particularly limited as long as the medium can dissolve the protein and can be used for biological tissue. Examples thereof include those generally usable in the field of the art, such as water, physiological saline, and a phosphate buffer.

The content rate of the protein in the protein solution is not particularly limited as long as the protein can be dissolved at the content rate. The content rate of the protein in the protein solution is, for example, preferably 0.5% by mass to 20% by mass, more preferably 2% by mass to 16% by mass, and even more preferably 4% by mass to 12% by mass.

In the step of preparing the protein solution, by preparing a protein as a material and dissolving the protein in an aqueous medium, the protein solution is prepared. The protein as a material may be in the form of powder or solid. As the protein solution, a protein solution may be prepared in advance and used.

The temperature at the time of preparing the protein solution is not particularly limited, and may be a commonly used temperature, for example, about 0°C to 60°C and preferably about 3°C to 30°C.

In addition, if necessary, the protein solution may contain components necessary for each step which will be described later, for example, a crosslinking agent, a component useful for adding predetermined properties to a substrate for regenerative medicine, and the like.

In order to obtain protein granules having voids, it is preferable to perform a step of cooling the protein solution to a temperature equal to or lower than an ice crystal forming temperature before the freeze-drying step.

In a case where the aforementioned step is performed, it is possible to obtain a protein-containing intermediate with ice crystals having an appropriate size that are on the inside of the intermediate. Due to the formed ice crystals, some of the peptide chains of the protein are densely aligned while others are sparsely aligned, and the protein-containing intermediate is solidified in this state. Therefore, after the ice crystals disappear, voids are formed in the protein-containing intermediate in the form of network. By performing the drying step which will be described later, the ice crystals can easily disappear. The pore size of the network in the protein-containing intermediate can be adjusted by the ice crystal temperature, the cooling time, or a combination of these.

The ice crystal forming temperature means a temperature at which at least a portion of the protein solution freezes. The ice crystal forming temperature varies with the concentration of solid contents including the protein in the protein solution. Generally, the ice crystal forming temperature can be equal to or lower than -10°C. The protein solution can be cooled preferably to -100°C to -10°C, more preferably to -80°C to -20°C, and even more preferably to -40°C to -60°C.

In view of easily generating uniform ice crystals, the time for which the protein solution is kept at a temperature equal to or lower than the ice crystal forming temperature is preferably 1 to 6 hours.

By treating the protein solution at the ice crystal forming temperature, a protein-containing intermediate having a network structure is obtained. In the present specification, the term "network structure" means a structure including a large amount of voids on the inside thereof, and is not limited to a planar structure. Furthermore, the network structure includes, as a structural portion, not only a fibrous structure but also a wall-like structure. In addition, the network structure means the structure of a material including a protein and does not include a structure on a molecular level such as collagen fiber.

"Having a network structure" means that voids having a size in micron order or a bigger size are formed by the wall-like structure constituted with the material including a protein. That is, "having a network structure" means having a pore size equal to or greater than 1 μm.

The shape of the network in the protein-containing intermediate is not particularly limited, and may be a two-dimensional structure such as a honeycomb or a three-dimensional structure such as a cancellous bone. The cross-sectional shape of the network may be a polygon, a circle, an ellipse, or the like. The three-dimensional shape of the network in the protein-containing intermediate may be columnar or spherical.

In the freeze-drying step, the protein solution is subjected to freeze-drying, thereby obtaining a freeze-dried material. In a case where the protein solution is subjected to the ice crystal formation treatment, the protein-containing intermediate may be directly subjected to the freeze-drying treatment.

As the freezing condition, the condition generally used for freeze-drying a protein may be used as it is. The freeze-drying can be performed, for example, for 0.5 hours to 300 hours. There is no particular limitation on the freeze dryer that can be used.

In the pulverization step, the freeze-dried material of a protein is pulverized, thereby obtaining a pulverized material. The obtained pulverized material can be incorporated into a tissue repair material as protein granules. For the pulverization, a pulverizer such as a hammer mill or a screen mill can be used. From the viewpoint of small variation in particle size distribution between tests, a screen mill (for example, Comil manufactured by Quadro Engineering) is preferable because this device makes it possible to collect materials pulverized in the same size as needed. As the condition of the pulverization, in order to maintain the structure of the surface of the pulverized material, a cutting method by crushing is preferable. Furthermore, in order to maintain the internal structure of the granules, it is preferable to adopt a method in which the granules are not strongly compressed during pulverization.

After the pulverization step, for the purpose of sizing, a classification step can be additionally performed. In a case where the classification step is performed, it is possible to obtain a pulverized material of a protein having a uniform particle size. For the classification, for example, it is preferable to use a sieve having an opening size of 300 μm to 710 μm.

After the pulverization step, it is preferable to additionally perform a crosslinking step of crosslinking the protein in the obtained pulverized material. As the method of crosslinking, for example, it is possible to use known methods such as thermal crosslinking, crosslinking by an enzyme, crosslinking using various chemical crosslinking agents, and ultraviolet (UV) crosslinking. As the method of crosslinking, crosslinking using a chemical crosslinking agent or thermal crosslinking is preferable. It is also preferable that the protein is caused to have a structure of a higher order by at least any one of hydrophobic interaction, hydrogen bonding, or ionic interaction, in addition to crosslinking (covalent bonding).

In a case where the crosslinking is performed by an enzyme, the enzyme is not particularly limited as long as it acts to crosslink biodegradable materials with each other. The crosslinking can be performed preferably using transglutaminase and laccase, and most preferably using transglutaminase.

In the present invention, at the time of treating the protein by using a crosslinking agent such as aldehydes or a condensing agent, the temperature for mixing the crosslinking agent with the protein is not particularly limited as long as the solution can be uniformly stirred. The temperature is preferably 0°C to 40°C, more preferably 0°C to 30°C, even more preferably 3°C to 25°C, still more preferably 3°C to 15°C, yet more preferably 3°C to 10°C, and particularly preferably 3°C to 7°C.

After the protein is mixed with the crosslinking agent and stirred, the temperature thereof can be raised. The reaction temperature is not particularly limited as long as the crosslinking proceeds. Considering the denaturation or degradation of the protein, the reaction temperature is substantially 0°C to 60°C, more preferably 0°C to 40°C, even more preferably 3°C to 25°C, still more preferably 3°C to 15°C, yet more preferably 3°C to 10°C, and particularly preferably 3°C to 7°C.

In the case of crosslinking method using a chemical crosslinking agent, it is more preferable that the crosslinking is performed using glutaraldehyde as a chemical crosslinking agent. In a case where the crosslinking method using a chemical crosslinking agent is adopted, the chemical crosslinking agent may be added to the protein solution such that the protein is crosslinked before the drying step.

The crosslinking temperature applied to the thermal crosslinking method is preferably 100°C to 200°C, more preferably 120°C to 170°C, and even more preferably 130°C to 160°C. In a case where the thermal crosslinking method is adopted, it is possible to avoid the use of a crosslinking agent.

The treatment time of the thermal crosslinking varies with the crosslinking temperature, the type of the protein, and the degree of degradation to be maintained. For example, in a case where CBE3, which is used in Examples that will be described later, is used as a protein, the thermal crosslinking conditions are as follows. In a case where the actual temperature is about 136°C, the treatment time is preferably 2 hours to 20 hours, more preferably 3 hours to 18 hours, and even more preferably 5 hours to 8 hours. In view of preventing oxidation, the thermal crosslinking treatment is preferably performed under reduced pressure or performed in a vacuum or in an inert gas atmosphere. The degree of pressure reduction is preferably equal to or lower than 4 hPa. Nitrogen is preferable as the inert gas, and from the viewpoint of uniform heating, crosslinking in an inert gas atmosphere is more preferable than crosslinking in a vacuum. The heating unit is not particularly limited, and examples thereof include a vacuum oven such as DP-43 manufactured by Yamato Scientific co., ltd., and the like.

The method for manufacturing the blocks preferably includes cooling a protein solution containing 7% by mass to 9% by mass of a protein for 1 hour to 6 hours at a temperature of -40°C to -60°C and then performing a freeze-drying treatment so as to obtain a freeze-dried material of a protein, pulverizing the freeze-dried material so as to obtain a pulverized material, and performing thermal crosslinking on the pulverized material for 3 hours to 7 hours in a nitrogen atmosphere at a temperature of 130°C to 160°C.

Furthermore, by sieving the obtained crosslinked blocks, granules having a desired size can be obtained. The blocks used in the present invention are preferably in the form of granules obtained as described above.

<Fibrin>

Fibrin is a protein involved in blood coagulation. Fibrinogen is released from platelets having undergone activation or the like, and from the fibrinogen, fibrin monomers are generated by the action of thrombin. The fibrin monomers form a polymer and are gelated, and as a result, fibrin clots are formed.

<Platelets>

The platelets having undergone activation or the like release fibrinogen. Platelet activation is known to occur, for example, by the bonding of thrombin to the platelets. In the present invention, it has been revealed that fibrinogen is released by the contact between blocks, which each include a protein having repetitive Arg-Gly-Asp sequences derived from human collagen, and platelets. In the present invention, through the contact between the aforementioned blocks and platelets, a fibrin composition is manufactured which includes the blocks each including a protein having repetitive Arg-Gly-Asp sequences derived from human collagen, fibrin, and platelets.

[Method for manufacturing fibrin composition]

The present invention also relates to a method for manufacturing a fibrin composition, including a step of mixing blocks, which each include a protein having repetitive Arg-Gly-Asp sequences derived from human collagen, with blood: and a step of subjecting the obtained mixture to centrifugation.

The blocks, which each include a protein having repetitive Arg-Gly-Asp sequences derived from human collagen, are as described above in the present specification.

The blood may be either whole blood or plasma, but between these, whole blood can be preferably used. In addition, the blood may be autologous or allogenic (blood from another donor), but is preferably autologous.

The mass ratio of the blocks to blood is not particularly limited, but is preferably 1:500 to 1:100, more preferably 1:400 to 1:100, and even more preferably 1:300 to 1:150.

The blocks and blood can be mixed together preferably in a plastic tube. The plastic tube will be described later. For example, by adding blood to a plastic tube preliminarily filled with the blocks each including a protein having repetitive Arg-Gly-Asp sequences derived from human collagen, the blocks and blood can be mixed together.

The mixture of the blocks and blood obtained as above is subjected to centrifugation. The conditions of centrifugation are not particularly limited as long as the fibrin composition including the blocks, fibrin, and platelets is formed. Generally, the centrifugation can be performed under centrifugal force which is $200 \times g$ to $1,000 \times g$ and preferably $400 \times g$ to $800 \times g$. The centrifugation time is not particularly limited, but is generally 3 minutes to 15 minutes, and preferably 5 minutes to 10 minutes. Centrifugation can be performed at room temperature (for example, 25°C). Note that $1 \times g$ equals $1.12 \times r \times (rpm/1,000)^2$. r represents a radius (mm), and rpm represents the a rotation speed per minute.

By the centrifugation described above, it is possible to manufacture a fibrin composition including blocks, which each include a protein having repetitive Arg-Gly-Asp sequences derived from human collagen, fibrin, and platelets.

[Substrate for regenerative medicine]

The fibrin composition according to the embodiment of the present invention can be used as a substrate for regenerative medicine.

The fibrin composition or the substrate for regenerative medicine according to the embodiment of the present invention can induce bone regeneration by being administered to a subject in need of bone regeneration (for example, mammals such as human beings).

The fibrin composition or the substrate for regenerative medicine according to the embodiment of the present invention can be used, for example, as a substrate for bone formation as a pretreatment for periodontal disease treatment or implant treatment.

The fibrin composition or the substrate for regenerative medicine according to the embodiment of the present invention can also be used as a substrate for bone regeneration treatment in the field of orthopedics.

Examples of diseases to be treated using the fibrin composition or the substrate for regenerative medicine according to the embodiment of the present invention include periodontal diseases, oral surgery diseases, traumatic bone injuries, osteoporosis, arthropathy, inflammatory diseases of muscle tendon, intractable mucocutaneous ulcer, dry eye, and the like.

The dosage, usage, and dosage form of the fibrin composition or the substrate for regenerative medicine according to the embodiment of the present invention can be appropriately determined according to the purpose of use. For example, the fibrin composition or the substrate for regenerative medicine according to the embodiment of the present invention may be administered to a target site in a living body directly or by means of injection, application, or the like after being suspended in a liquid excipient such as distilled water for injection, physiological saline for injection, or an aqueous solvent like a buffer having a pH of 5 to 8 (for example, a buffer based on phosphoric acid or citric acid). Alternatively, the composition or the substrate may be mixed with an appropriate excipient so as to form an ointment, gel, cream or the like, and then applied.

Preferably, the fibrin composition or the substrate for regenerative medicine according to the embodiment of the present invention can be directly administered to a bone defect site in a living body.

The dosage of the fibrin composition or the substrate for regenerative medicine according to the embodiment of the present invention is not particularly limited. For example, the dosage thereof per surface area of 1 cm$^2$ of a living body to be administered with the composition or the substrate is 1 mg to 100 mg, preferably 10 mg to 100 mg, and more preferably 10 mg to 30 mg.

[Kit]

According to the present invention, there is provided a kit for being used in the method for manufacturing a fibrin composition according to the embodiment of the present invention. The kit includes blocks, which each include a protein having repetitive Arg-Gly-Asp sequences derived from human collagen, and a plastic tube.

The details of the blocks, which each include a protein having repetitive Arg-Gly-Asp sequences derived from human collagen, are as described above in the present specification.

The plastic tube is a bottomed tubular container made of plastic.

As the plastic tube, a plastic blood collection tube is preferably used, and a vacuum blood collection tube may also be used. In a vacuum blood collection system using a vacuum blood collection tube, the internal pressure of an airtight tube made of plastic is reduced in advance, and blood is collected using a double tipped needle. In a case where the blood collection needle is stabbed into a blood vessel and also stabbed into the tube through the other tip, because the internal pressure of the tube has been reduced, blood is drawn into the tube.

The vacuum blood collection tube is a bottomed tubular container having a sealing member at an opening portion. Specifically, the vacuum blood collection tube is constituted with a bottomed tubular container that is open at one end and closed at the other end, and a sealing member having a structure that seals the opening portion of the bottomed tubular container and allows a puncture needle of a blood collection device to penetrate the sealing member. The internal pressure of the vacuum blood collection tube has been reduced.

The plastic tube has a dimension in which, for example, an outside diameter is 10 to 20 mm, a length is 60 to 170 mm, and a wall thickness is about 0.5 mm to 2 mm.

The material of the plastic tube is not particularly limited, and examples thereof include polyester such as polypropylene and polyethylene terephthalate, an acrylic resin, and polycarbonate. As the material of the plastic tube, plastic having high gas barrier properties is preferable, and for example, polyester is more preferable.

It is preferable that the plastic tube does not include a serum (plasma) separating agent.

The present invention will be more specifically described based on the following examples, but the present invention is not limited to the examples.

Examples

[Reference Example 1] Recombinant peptide (recombinant gelatin)

As a recombinant peptide (recombinant gelatin), the following CBE3 was prepared (described in WO2008/103041A).

CBE3:

Molecular weight: 51.6 kD
Structure: GAP[(GXY)$_{63}$]$_3$G
Number of amino acids: 571
RGD sequences: 12
Imino acid content: 33%

Almost 100% of the amino acids are GXY repeating structures. The amino acid sequence of CBE3 does not include serine, threonine, asparagine, tyrosine, and cysteine. CBE3 has an ERGD sequence.
Isoelectric point: 9.34
GRAVY value: -0.682
1/IOB value: 0.323

Amino acid sequence (SEQ ID NO: 1 in Sequence Listing) (same as SEQ ID NO: 3 in WO2008/103041A, X at the end is modified to "P")

GAP(GAPGLQGAPGLQGMPGERGAAGLPGPKGERGDAGPKGADGAPGAPGL

QGMPGERGAAGLPGPKGERGDAGPKGADGAPGKDGVRGLAGPIGPPGERGAAGLPG

PKGERGDAGPKGADGAPGKDGVRGLAGPIGPPGPAGAPGAPGLQGMPGERGAAGLP

GPKGERGDAGPKGADGAPGKDGVRGLAGPP)$_3$G

[Reference Example 2] Preparation of granules of porous recombinant peptide

An aluminum cylindrical cup-shaped container having a thickness of 5 mm and a diameter of 98 mm was prepared. In a case where the cylindrical cup has a curved surface as lateral surface, the lateral surface is closed with 1 mm aluminum, and the bottom surface (flat circular shape) thereof is also closed with 5 mm aluminum. On the other hand, the top surface is open. Furthermore, only the inside of the lateral surface is uniformly lined with Teflon (registered trademark) having a thickness of 3 mm. As a result, the inside diameter of the cylindrical cup becomes 90 mm. Hereinafter, this container will be referred to as cylindrical container.

Approximately 18 ml of an aqueous gelatin solution including 7.5% by mass of recombinant gelatin was poured into the cylindrical container, and then left in a freezer (Hitachi, ultra-low temperature freezer RS-U30T) at -50°C for 1 hour or longer, thereby obtaining a frozen gelatin block. This gelatin block was freeze-dried (TAKARA Co., Ltd, TF5-85ATNNNN) and pulverized using a pulverizer (Quadro Engineering, Comil U10) through a screen having a pore size of about 1 mm, and a fraction under a sieve having an opening size of 1.4 mm was collected. The obtained gelatin granules were treated for 4.75 hours in a nitrogen atmosphere at 135°C (Yamato Scientific co., ltd., DP-43), thereby obtaining porous granules.

[Example 1: Influence of plastic blood collection tube on PRF clot formation]

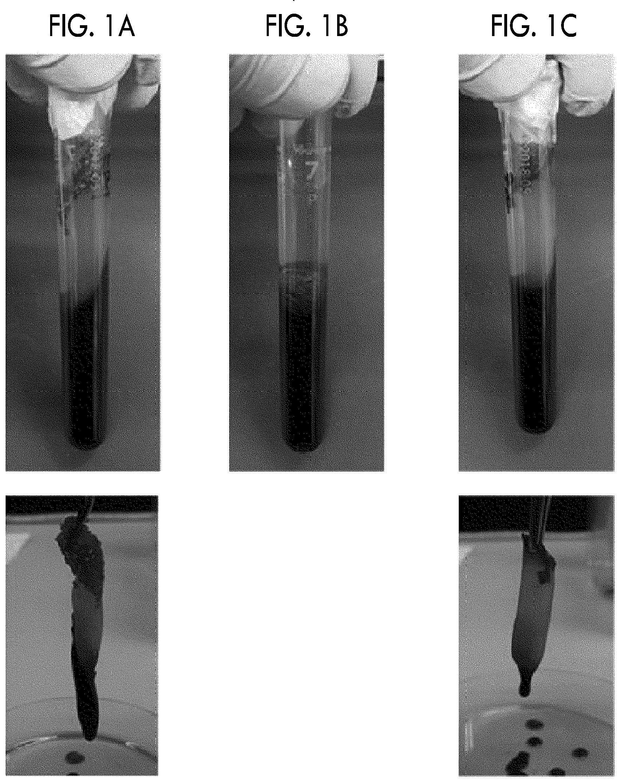

The porous granules obtained in Reference Example 2 were pulverized using a pulverizing mill (TSUTSUI SCIENTIFIC INSTRUMENTS CO., LTD., MICRO VIBRO SIFTER (M-3T)). The granules were intermittently pulverized three times for 50 seconds in total. The obtained pulverized material was passed through a stainless steel test sieve having a nominal opening size of 710 μm described in Appendix Table 2 of Japanese Industrial Standard JIS Z8801-1. Furthermore, granules (hereinafter, referred to as FBG as well) remaining on a stainless steel test sieve having a nominal opening size of 500 μm were collected and used for the following experiments.
The porosity of the granules was 80% to 90%. The porosity was measured as described above in the present specification.
FBG described above or Terudermis (registered trademark) (manufactured by Olympus-Terumo Biomaterials Corp) (shredded in a size of about 1 x 1 x 1 mm) was added to a plain plastic blood collection tube (Venoject (registered trademark) II manufactured by Terumo Corporation), thereby preparing a blood collection tube. To each plastic blood collection tube, FBG was added in an amount of 22 mg to 28 mg (25 mg on average), and Terudermis (registered trademark) was added in an amount of 13 mg to 22 mg (17 mg on average).
Fresh venous blood collected using a plastic blood collection tube was dispensed into these three types of plastic blood collection tubes, mixed, and then set in a centrifuge (trade name: Medifuge). The amount of blood collected into each plastic blood collection tube was about 7 mL. Centrifugation was performed for 8 minutes at 25°C under a centrifugation condition of 1,100xg. Fig. 1A to 1C show the states of the obtained fibrin compositions observed with the unaided eye. Fig. 1A shows a case where FBG was added to a plastic blood collection tube, Fig. 1B shows a case where a plastic blood collection tube to which FBG or Terudermis was not added was used, and Fig. 1C shows a case where Terudermis (registered trademark) was added to a plastic blood collection tube.
As shown in Fig. 1A to 1C, in the plastic blood collection tube to which FBG or Terudermis was not added, the formation of PRF clots was not confirmed, but in a case where FBG or Terudermis (registered trademark) was added and mixed by inversion and then subjected to centrifugation, the formation of PRF clots was confirmed.

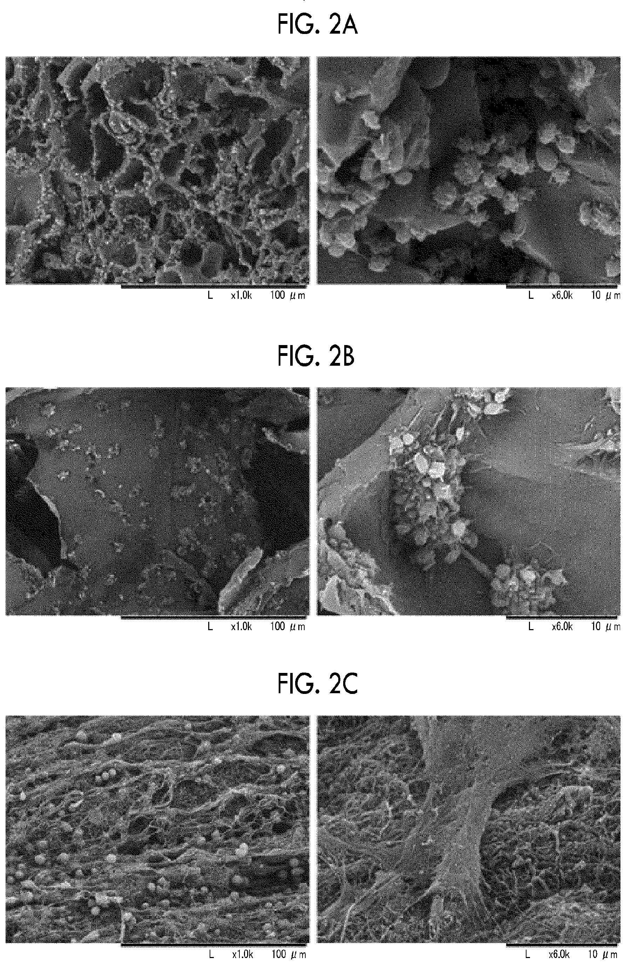

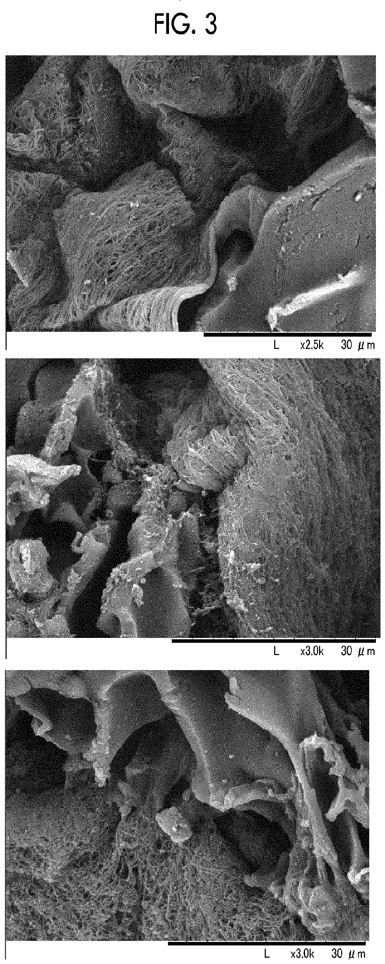

Figs. 2A to 2C and Fig. 3 show images of the obtained fibrin composition captured using a scanning electron microscope (SEM).
Fig. 2 shows the microstructures of the complex with FBG (Fig. 2A), the complex with Terudermis (registered trademark) (Fig. 2B), and the PRF clots to which FBG or Terudermis was not added (Fig. 2C). Although both the FBG and Terudermis (registered trademark) adhered to platelets (spherical objects having a diameter of several microns), Terudermis (registered trademark) has been found to more strongly cause platelet aggregation. The formation of fibrin fibers was confirmed outside these visual fields, that is, where there is no each substrate. On the other hand, in a case where only PRF clots are used, platelets are found to be incorporated into the fibrin network together with white blood cells.
Fig. 3 shows the microstructures of the complexes with FBG. Fibrin fibers are found to be formed around FBG.
In Figs. 2A to 2C and Fig. 3, it has been confirmed that a fibrin composition including blocks, which each include a protein having repetitive Arg-Gly-Asp sequences derived from human collagen, fibrin, and platelets is manufactured.

[Example 2: Comparison of bone regeneration effect in cranial bond-deficient mouse models]

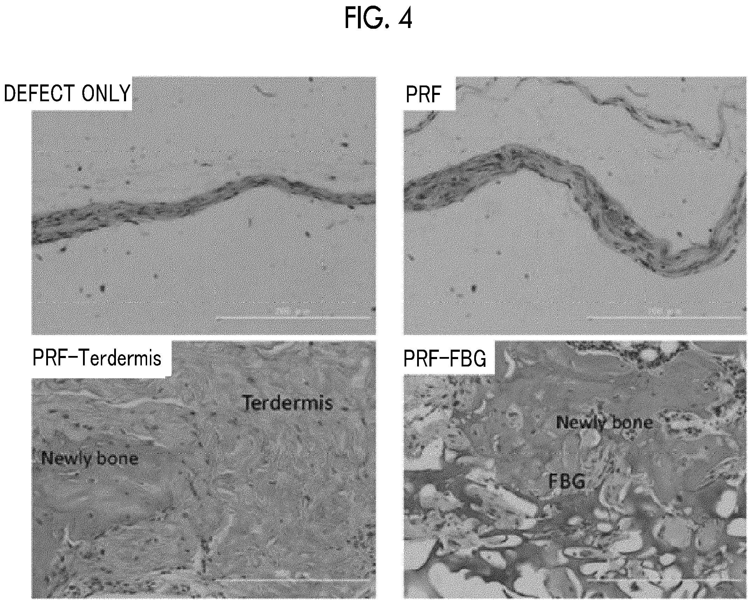

In order to compare the bone regeneration activity of the complex of FBG and PRF, a defect having a diameter of 3 mm was created in the cranial bone of immunodeficient hairless mice (ICR hairless mice, male, 6 to 8 weeks old). By using these mice, bone regeneration was evaluated under four conditions of (1) no treatment, (2) transplanting PRF alone, (3) transplanting a complex of PRF + Terudermis, and (4) transplanting a complex of PRF + FBG. Fig. 4 shows the histopathological findings revealed 4 weeks after the treatment.
From the findings shown in Fig. 4, it has been confirmed that in a case where PRF is transplanted alone, PRF does not stay for 4 weeks at the site of transplantation, undergoes biodegradation at an early stage, and substantially does not cause bone regeneration. On the other hand, it has been confirmed that in a case where PRF is combined with Terudermis (registered trademark) or FBG, although the presence of PRF itself cannot be clearly confirmed from the findings by hematoxylin·eosin (HE) staining, bone regeneration (formation of new bone) is clearly observed. In addition, it has been confirmed that compared to Terudermis (registered trademark), FBG more markedly induces the formation of new bone.
Table 1 shows the area of new bone formed at the site of transplantation.
By using the image processing software Image J, the formed bone and other tissues were binarized from histopathological images of the site of transplantation so as to create images, and the area of the formed bone was calculated. The area of bone formed by FBG has been found to be about 18 times larger than the area of bone formed by PRF alone and about 6 times larger than the area of bone formed by Terudermis (registered trademark).

Table 1]

| | Untreated | Treated only with PRF | PRF + Terudermis | PRF + FBG |
|---|---|---|---|---|
| Area of newly formed bone ($\mu$m$^2$, average) | 11223.34 | 11563.55 | 35988.14 | 207866.00 |

[Summary of Examples]

From the above findings, it has been revealed that in a case where FBG is added to a plastic blood collection tube, PRF clots can be formed as in a case where a glass blood collection tube is used, and the complex of PRF + FBG has a potent bone regeneration activity. Therefore, it has been revealed that a PRF complexation technique using a protein, which has repetitive Arg-Gly-Asp sequences derived from human collagen, not only enables a plastic blood collection tube to substitute a glass blood collection tube, but also can bring about therapeutic effects that are equal to or stronger than therapeutic effects exhibited in a case where PRF is used alone.

[Sequence list] PCT_fibrin composition, re_20190128_145216_1.txt

**Claims**

1. A fibrin composition: comprising:

   blocks which each include a protein having repetitive Arg-Gly-Asp sequences derived from human collagen;
   fibrin; and
   platelets.

2. The fibrin composition according to claim 1,
   wherein the blocks are in the form of granules.

3. The fibrin composition according to claim 1 or 2,
   wherein the blocks are in the form of granules obtained by sieving blocks obtained by pulverizing a porous material of the protein.

4. The fibrin composition according to claim 2 or 3,
   wherein the granules have such a size that the granules pass through a 1,000 μm sieve and remain on a 100 μm sieve.

5. The fibrin composition according to claim 2 or 3,
   wherein the granules have such a size that the granules pass through a 710 μm sieve and remain on a 500 μm sieve.

6. The fibrin composition according to any one of claims 2 to 5,
   wherein a porosity of the granules is 70% to 95%.

7. The fibrin composition according to any one of claims 1 to 6,
   wherein the protein is a recombinant peptide.

8. The fibrin composition according to claim 7,
   wherein the recombinant peptide is represented by the following formula,

   Formula:          A-[(Gly-X-Y)n]m-B

   in the formula, A represents any amino acid or amino acid sequence, B represents any amino acid or amino acid sequence, n pieces of X each independently represent any amino acid, n pieces of Y each independently represent any amino acid, n represents an integer of 3 to 100, m represents an integer of 2 to 10, and n pieces of Gly-X-Y may be the same as or different from each other.

9. The fibrin composition according to claim 7 or 8,

   wherein the recombinant peptide is any of
   a peptide consisting of an amino acid sequence described in SEQ ID NO: 1;
   a peptide having an amino acid sequence, which is obtained by the deletion, substitution, or addition of one or several amino acids in the amino acid sequence described in SEQ ID NO: 1, and having biocompatibility; or
   a peptide having an amino acid sequence, which shares sequence identity equal to or higher than 80% with the amino acid sequence described in SEQ ID NO: 1, and having biocompatibility.

10. The fibrin composition according to any one of claims 1 to 9,
    wherein in the blocks, the protein is crosslinked by heat, ultraviolet rays, or an enzyme.

11. A substrate for regenerative medicine, comprising:
    the fibrin composition according to any one of claims 1 to 10.

12. A method for manufacturing a fibrin composition, comprising:

    a step of mixing blocks, which each include a protein having repetitive Arg-Gly-Asp sequences derived from human collagen, with blood; and
    a step of subjecting the obtained mixture to centrifugation.

13. The method according to claim 12,
    wherein the blocks are in the form of granules.

14. The method according to claim 12 or 13,
    wherein the blocks are in the form of granules obtained by pulverizing a porous material of the protein.

15. The method according to claim 13 or 14,
    wherein the granules have such a size that the granules pass through a 1,000 $\mu$m sieve and remain on a 100 $\mu$m sieve.

16. The method according to claim 13 or 14,
    wherein the granules have such a size that the granules pass through a 710 $\mu$m sieve and remain on a 500 $\mu$m sieve.

17. The method according to any one of claims 13 to 16,
    wherein a porosity of the granules is 70% to 95%.

18. The method according to any one of claims 12 to 17,
    wherein the protein is a recombinant peptide.

19. The method according to claim 18,
    wherein the recombinant peptide is represented by the following formula,

    Formula:          A-[(Gly-X-Y)n]m-B

    in the formula, A represents any amino acid or amino acid sequence, B represents any amino acid or amino acid sequence, n pieces of X each independently represent any amino acid, n pieces of Y each independently represent any amino acid, n represents an integer of 3 to 100, m represents an integer of 2 to 10, and n pieces of Gly-X-Y may be the same as or different from each other.

20. The method according to claim 18 or 19,

    wherein the recombinant peptide is any of
    a peptide consisting of an amino acid sequence described in SEQ ID NO: 1;
    a peptide having an amino acid sequence, which is obtained by the deletion, substitution, or addition of one or several amino acids in the amino acid sequence described in SEQ ID NO: 1, and having biocompatibility; or
    a peptide having an amino acid sequence, which shares sequence identity equal to or higher than 80% with the amino acid sequence described in SEQ ID NO: 1, and having biocompatibility.

**21.** The method according to any one of claims 12 to 20,
wherein in the blocks, the protein is crosslinked by heat, ultraviolet rays, or an enzyme.

**22.** The method according to any one of claims 12 to 21,
wherein the blood is whole blood.

**23.** The method according to any of claims 12 to 22,
wherein a mass ratio of the blocks to the blood is 1:500 to 1:100.

**24.** A kit for being used in the method for manufacturing a fibrin composition according to any one of claims 12 to 23,
the kit comprising:

blocks which each include a protein having repetitive Arg-Gly-Asp sequences derived from human collagen; and
a plastic tube.

FIG. 1A

FIG. 1B

FIG. 1C

## FIG. 2A

## FIG. 2B

## FIG. 2C

## FIG. 3

L    x2.5k    30 μm

L    x3.0k    30 μm

L    x3.0k    30 μm

FIG. 4

## INTERNATIONAL SEARCH REPORT

| | International application No. |
|---|---|
| | PCT/JP2019/002869 |

**A. CLASSIFICATION OF SUBJECT MATTER**
Int.Cl. A61L27/22(2006.01)i, A61C8/00(2006.01)i, A61L27/24(2006.01)i,
C07K14/75(2006.01)n, C07K14/78(2006.01)n, C12N15/12(2006.01)n,
C12P21/02(2006.01)n
According to International Patent Classification (IPC) or to both national classification and IPC

**B. FIELDS SEARCHED**

Minimum documentation searched (classification system followed by classification symbols)
Int.Cl. A61L27/22, A61C8/00, A61L27/24, C07K14/75, C07K14/78, C12N15/12,
C12P21/02

Documentation searched other than minimum documentation to the extent that such documents are included in the fields searched
Published examined utility model applications of Japan     1922–1996
Published unexamined utility model applications of Japan   1971–2019
Registered utility model specifications of Japan           1996–2019
Published registered utility model applications of Japan   1994–2019

Electronic data base consulted during the international search (name of data base and, where practicable, search terms used)
JSTPlus/JMEDPlus/JST7580(JDreamIII), CAplus/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2004-123576 A (MEDGEL CORP.) 22 April 2004, claims, paragraphs [0011], [0013]-[0018], examples, etc.<br>(Family: none) | 1-6, 10, 11<br>7-11 |
| X<br>Y | WO 2011/048803 A1 (NITTO DENKO CORPORATION) 28 April 2011, claims, paragraphs [0035], [0041], examples, etc.<br>& US 2012/0237586 A1, claims, paragraphs [0050], [0055], examples & EP 2491958 A1 | 1-6, 10, 11<br>7-11 |

☒ Further documents are listed in the continuation of Box C.   ☐ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>28.02.2019 | Date of mailing of the international search report<br>12.03.2019 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

| International application No. |
| --- |
| PCT/JP2019/002869 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2010-519251 A (FUJIFILM MANUFACTURING EUROPE B.V.) 03 June 2010, paragraph [0022] & WO 2008/103041 A1, page 6, line 4 & US 2010/0062531 A1 & EP 1961414 A1 | 1-11 |
| Y | WO 2011/108537 A1 (FUJIFILM CORPORATION) 09 September 2011, claims, paragraphs [0046]-[0049], examples, etc. & US 2013/0004549 A1, claims, paragraphs [0080]-[0083], examples & EP 2543398 A1 | 1-24 |
| Y | WO 2011/108517 A1 (FUJIFILM CORPORATION) 09 September 2011, claims, paragraphs [0045]-[0048], examples, etc. & US 2012/0329157 A1, claims, paragraphs [0076]-[0079], examples & EP 2543397 A1 | 1-24 |
| Y | JP 2005-160669 A (OLYMPUS CORPORATION) 23 June 2005, claims, paragraphs [0025], [0033], examples, etc. (Family: none) | 1-11 |
| Y | JP 2004-505747 A (SYNTHES AKTIENGESELLSCHAFT) 26 February 2004, claims, examples, etc. & WO 2002/015950 A1, claims, examples & US 2005/0074433 A1 & EP 1311308 A1 | 1-24 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2015167606 A
- WO 2012102094 A
- JP 2016528225 A
- JP 2014531467 A
- EP 1014176B A
- US 6992172 B
- WO 2004085473 A
- WO 2008103041 A
- EP 1014176 A2

### Non-patent literature cited in the description

- Phitogr. Gelatin 2. PJ Academic
- *J. Mol. Biol.,* 1990, vol. 215, 403-410
- *Pharmaceutical Bulletin,* 1954, vol. 2 (2), 163-173
- *Scope of Chemistry,* 1957, vol. 11 (10), 719-725
- *Fragrance Journal,* 1981, vol. 50, 79-82
- **YOSHIKI KODA et al.** New Edition of Organic Conception Diagram-Fundamentals and Applications. SANKYO SHUPPAN Co., Ltd, 2008
- Bairoch A.;Protein Identification and Analysis Tools on the ExPASy Server. **GASTEIGER E. ; HOOGLAND C. ; GATTIKER A. ; DUVAUD S. ; WILKINS M.R. ; APPEL R.D.** The Proteomics Protocols Handbook. Humana Press, 2005, 571-607
- **GASTEIGER E. ; GATTIKER A. ; HOOGLAND C. ; IVANYI I. ; APPEL R.D. ; BAIROCH A.** ExPASy: the proteomics server for in-depth protein knowledge and analysis. *Nucleic Acids Res.,* 2003, vol. 31, 3784-3788
- The Japanese Pharmacopoeia